(19) Europäisches Patentamt
European Patent Office
Office européen des brevets

(11) Publication number: **0 113 108**
**B1**

(12) **EUROPEAN PATENT SPECIFICATION**

(45) Date of publication of patent specification: **29.07.87**

(51) Int. Cl.$^4$: **C 07 C 155/00**, A 01 N 47/12

(21) Application number: **83112991.1**

(22) Date of filing: **22.12.83**

(54) **Benzamidine group compounds and agricultural fungicidal compositions containing the same.**

(30) Priority: **28.12.82 JP 227468/82
28.12.82 JP 227469/82
28.12.82 JP 227470/82**

(43) Date of publication of application:
**11.07.84 Bulletin 84/28**

(45) Publication of the grant of the patent:
**29.07.87 Bulletin 87/31**

(84) Designated Contracting States:
**DE FR GB IT NL**

(56) References cited:
**GB-A-2 042 528**

(73) Proprietor: **Ube Industries, Ltd.
12-32, Nishihonmachi 1-chome
Ube-shi, Yamaguchi-ken (JP)**

(72) Inventor: **Nagai, Hirofumi c/o Central Research Laboratories
Ube Industries, Ltd. 1978-5, Oaza Kogushi
Ube-shi Yamaguchi-ken (JP)**
Inventor: **Yorie, Takashi c/o Central Research Laboratories
Ube Industries, Ltd. 1978-5, Oaza Kogushi
Ube-shi Yamaguchi-ken (JP)**
Inventor: **Iida, Seiji c/o Central Research Laboratories
Ube Industries, Ltd. 1978-5, Oaza Kogushi
Ube-shi Yamaguchi-ken (JP)**

(74) Representative: **Hansen, Bernd, Dr.rer.nat. et al
Hoffmann, Eitle & Partner Patentanwälte
Arabellastrasse 4
D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 113 108 B1**

**Description**

This invention relates to a novel benzamidine group compound and also to a novel agricultural fungicidal composition containing the same as an active ingredient.

Conventionally, various kinds of organomercury compounds, arsenic compounds, copper compounds and the like have frequently been employed for agricultural fungicides. However, these compounds have been revealed to have serious disadvantages such as residual pollution of soil, and toxicity or residual toxicity against man and beast, the use thereof has been limited. Furthermore, there have recently appeared drug-resistant fungi or bacteria in plant pests of various field crops, particularly in powdery mildew, gray mold, blast disease and sclerotium disease. Therefore, the effect on such plant pests by chemicals which have been employed for many years has been decreasing.

In GB—A—2 042 528 benzamidine derivatives having the formula

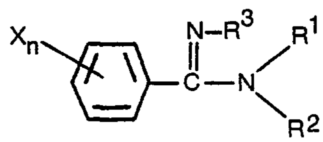

are disclosed as agricultural fungicides. These compounds encompass benzamidine compounds in which n represents 0, $R^1$ and $R^2$ represent an ethyl group and $R^3$ represents groups of the formulae

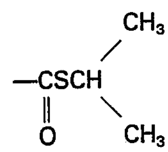 and 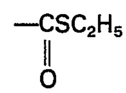

These compounds are excluded from the protection in the present patent.

For the reasons mentioned above, it has been desired to develop a new type of agricultural fungicides.

As a result of the extensive studies on fungicidal effect of various compounds for the purpose of developing a new agricultural fungicide, the present inventors have succeeded to synthesize a new benzamidine group compound and have found that they have an excellent fungicidal effect against plant pests parasitic on various field crops, and have accomplished this invention.

An object of this invention is to provide a novel benzamidine group compound.

Another object of this invention is to provide an agricultural fungicidal composition containing said benzamidine group compound as an active ingredient.

The novel compound according to this invention is a benzamidine group compound represented by the general formula (I):

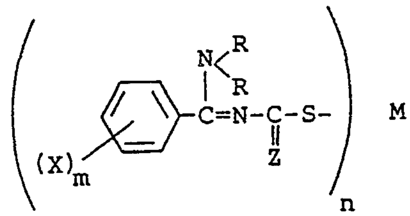 (I)

wherein R represents a $C_1$ to $C_4$ alkyl group; X represents a halogen atom or a $C_1$ to $C_4$ alkyl group; m represents an integer of 0, 1 or 2; Z represents an oxygen atom or a sulfur atom; n represents an integer of 1 or 2; when n is 1, M represents a $C_1$ to $C_4$ alkyl group, a $C_3$ to $C_5$ alkenyl group, a phenyl group of the formula

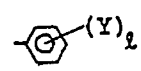

or a benzyl group of the formula

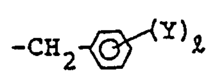

(in which Y represents a $C_1$ to $C_4$ alkyl group, a halogen atom, a $C_1$ to $C_4$ alkoxy group or a nitro group and l represents an integer of 0, 1, 2 or 3); and when n is 2, M represents a metal selected from nickel, cobalt and zinc; with the proviso that when Z is oxygen, M is not a $C_1$ to $C_4$ alkyl group.

More specifically, in a preferred embodiment, a benzamidine compound according to this invention is represented by the general formula (II):

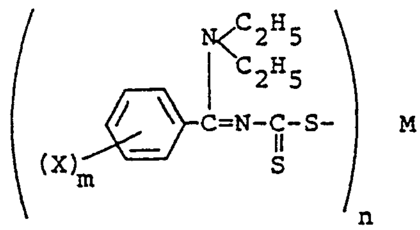

(II)

wherein X, *m* and *n*, have the same meanings as defined in the formula (I), and when n is 1, M represents a $C_1$ to $C_4$ alkyl group, a $C_3$ to $C_5$ alkenyl group, a benzyl group or a halogenated benzyl group and when n is 2, M has the same meaning as defined in the formula (I).

In the above general formula, the $C_1$ to $C_4$ alkyl group represented by X or M, is an alkyl group such as methyl, ethyl, n-(or i-)propyl and n-(i-, or tert-)butyl.

As the halogen atom represented by X or Y (a substituent on the benzyl group of the formula

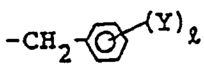

represented by M), there may be mentioned any of chlorine, bromine, fluorine and iodine.

The $C_3$ to $C_6$ alkenyl group represented by M is allyl, butenyl or pentenyl.

In another preferred embodiment, a benzamidine compound according to this invention is represented by the general formula (III):

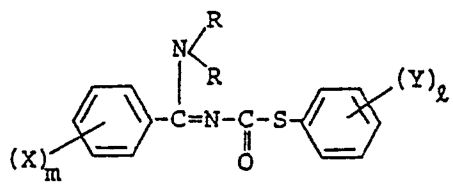

(III)

wherein R, X, m and l have the same meanings as defined in the formula (I) and Y represents a $C_1$ to $C_4$ alkyl group or a halogen atom.

In the above general formula, the $C_1$ to $C_4$ alkyl group represented by R, X or Y is methyl, ethyl, n-(or i-)propyl or n-(i- or tert-)butyl.

As the halogen atom represented by X or Y, there may be mentioned any of chlorine, bromine, fluorine and iodine.

In still another preferred embodiment, a benzamidine compound according to this invention is represented by the general formula (IV):

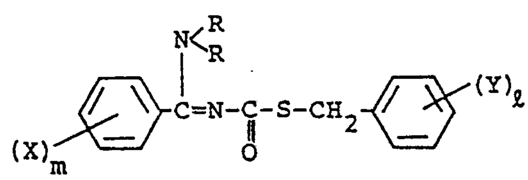

(IV)

wherein R, X, m and Y have the same meanings as defined in the formula (I) and l represents an integer of 0, 1 or 2.

In the above general formula, the $C_1$ to $C_4$ alkyl group represented by R, X or Y is methyl, ethyl, n-(or i-)propyl or n-(i- or tert-)-butyl.

As the halogen atom represented by X or Y, there may be mentioned any of chlorine, bromine, fluorine and iodine.

The $C_1$ to $C_4$ alkoxy group represented by Y is methoxy, ethoxy, propoxy and butoxy.

Examples of the benzamidine compounds having the abovementioned general formula (I) according to this invention are listed in Table 1.

TABLE 1

| Compound No. | Structural formula | Physical property |
|---|---|---|
| 1 | 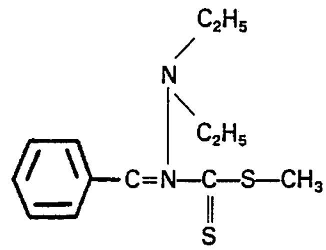 | $n_D^{27}$ 1.640 |
| 2 | 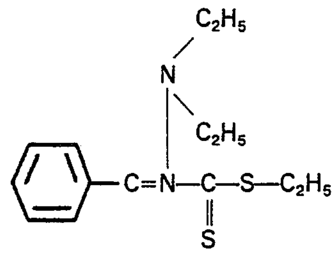 | $n_D^{20}$ 1.634 |
| 3 | 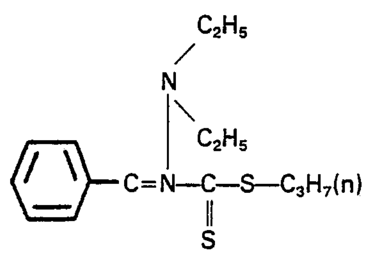 | $n_D^{28}$ 1.6160 |
| 4 | 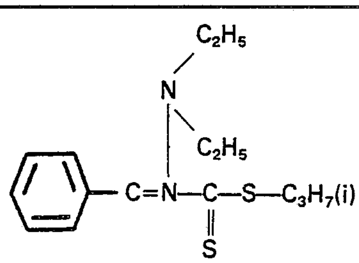 | $n_D^{26}$ 1.6161 |
| 5 | 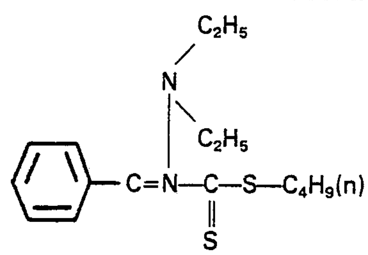 | $n_D^{26}$ 1.6102 |
| 6 | 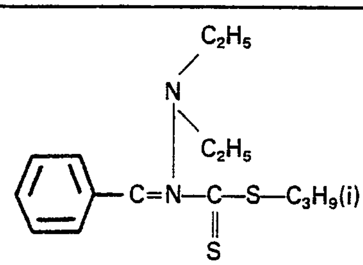 | $n_D^{26}$ 1.6082 |

TABLE 1 (continued)

| Compound No. | Structural formula | Physical property |
|---|---|---|
| 7 | 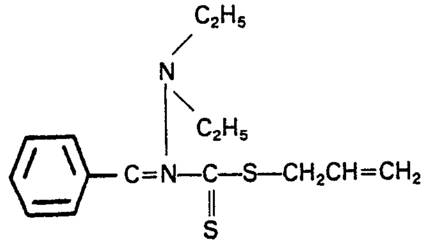 | $n_D^{26}$ 1.6320 |
| 8 | 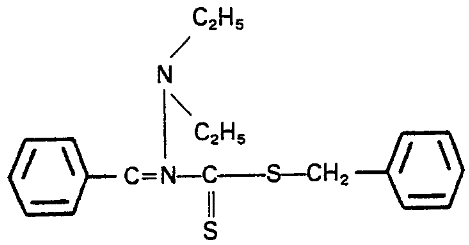 | $n_D^{27}$ 1.6540 |
| 9 | 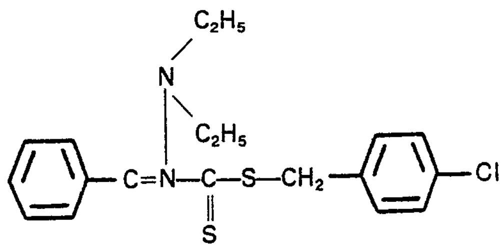 | $n_D^{27}$ 1.6478 |
| 10 | 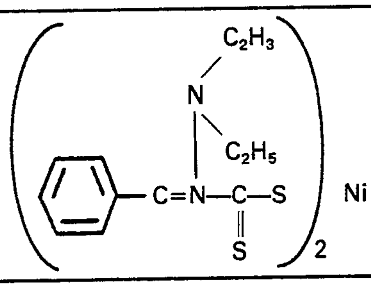 | dec. 184 ~ 189°C |
| 11 | 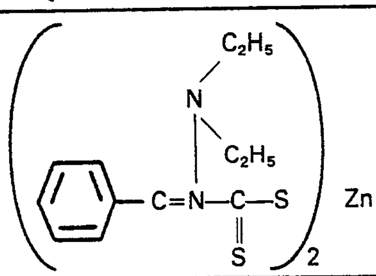 | m.p. 98 ~ 98°C |
| 12 | 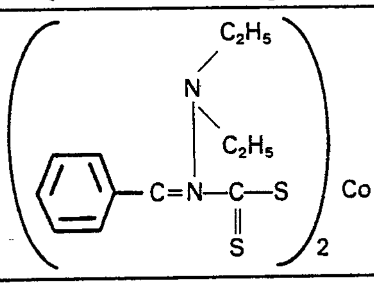 | dec. 204°C |

TABLE 1 (continued)

| Compound No. | Structural formula | Physical property |
| --- | --- | --- |
| 13 | 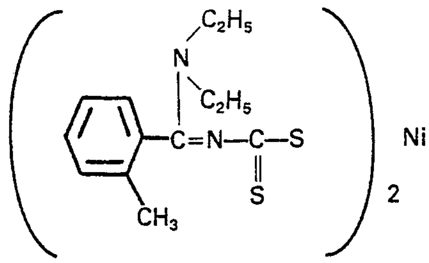 | dec. 215 ~ 220°C |
| 14 | 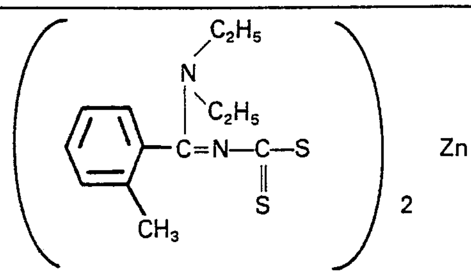 | m.p. 89 ~ 95°C |
| 15 | 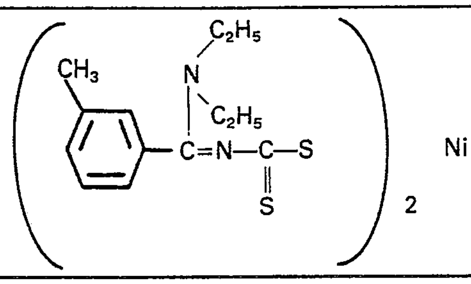 | dec. 200°C |
| 16 | 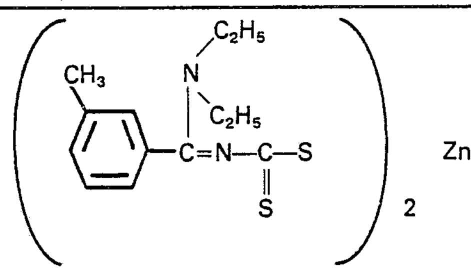 | dec. 115°C |
| 17 | 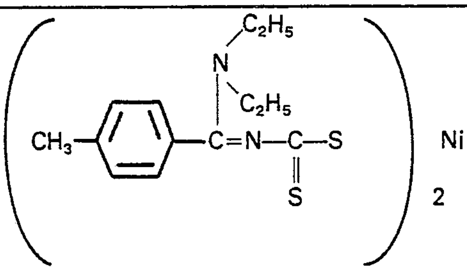 | dec. 200°C |
| 18 | 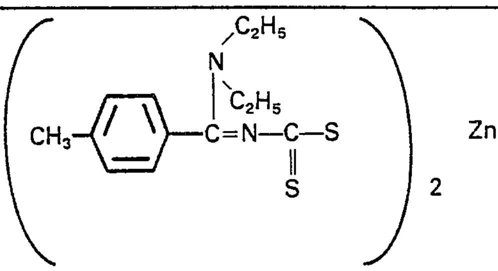 | dec. 118°C |

TABLE 1 (continued)

| Compound No. | Structural Formula | Physical property |
|---|---|---|
| 19 | 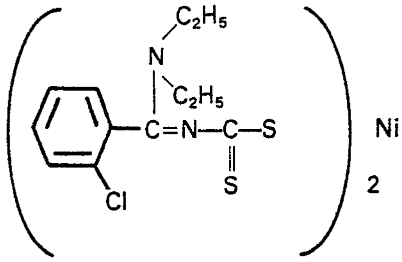 | dec. 205°C |
| 20 | 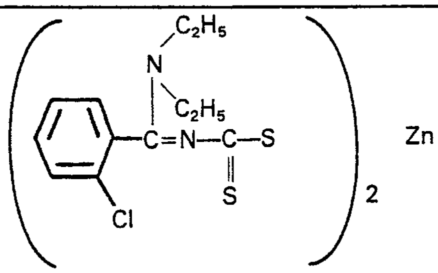 | dec. 110°C |
| 21 | 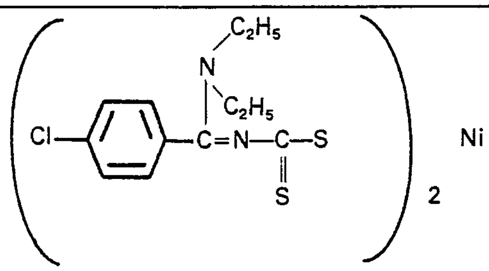 | dec. 200°C |
| 22 | 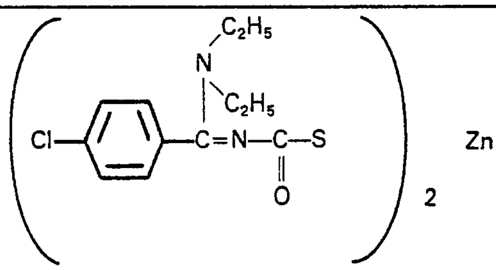 | dec. 120°C |
| 23 | 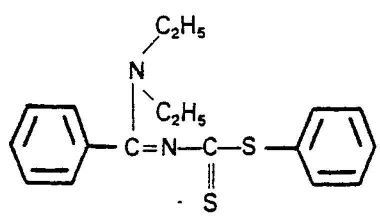 | m.p. 63—65°C |
| 24 | 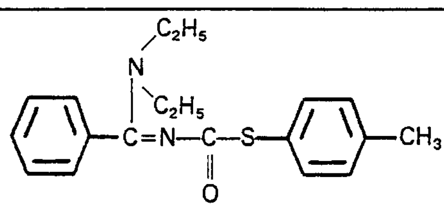 | m.p. 55-57°C |

TABLE 1 (continued)

| Compound No. | Structural Formula | Physical property |
|---|---|---|
| 25 | 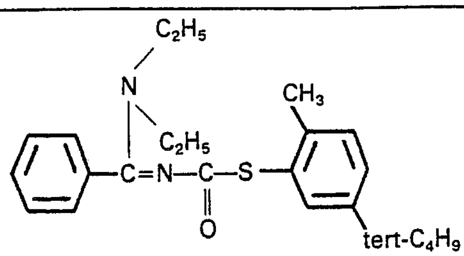 | $n_D^{28}$ 1.5626 |
| 26 | 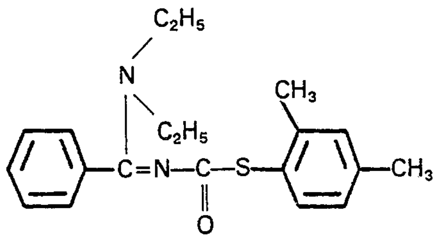 | m.p. 80 ~ 83°C |
| 27 | 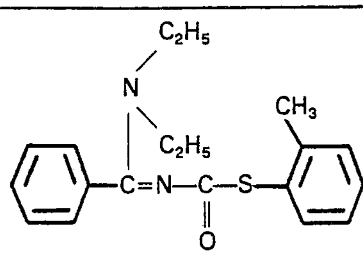 | $n_D^{20}$ 1.5998 |
| 28 | 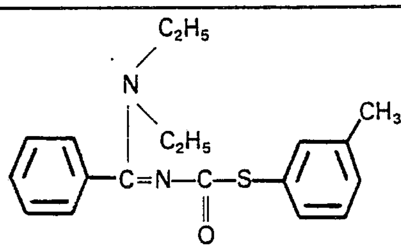 | $n_D^{29}$ 1.5992 |
| 29 | 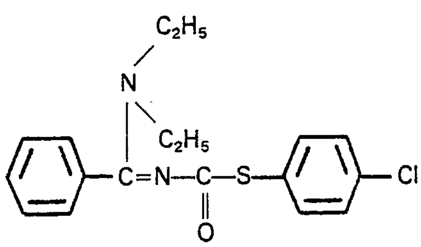 | m.p. 72 ~ 74°C |
| 30 | 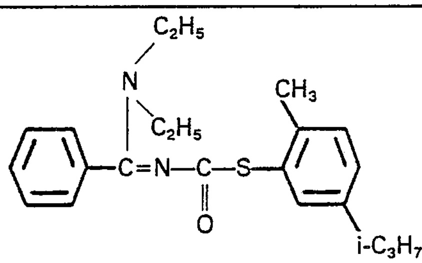 | m.p. 105 ~ 107°C |

TABLE 1 (continued)

| Compound No. | Structural Formula | Physical property |
|---|---|---|
| 31 | 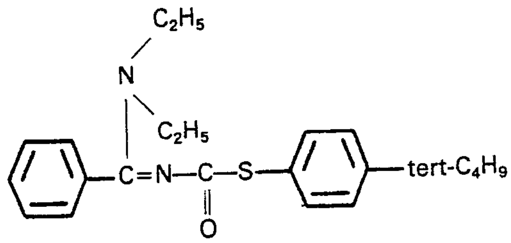 | $n_D^{28}$ 1.5885 |
| 32 | 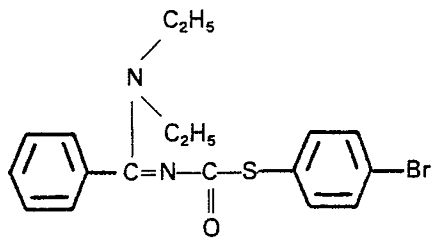 | m.p. 82 ~ 84°C |
| 33 | 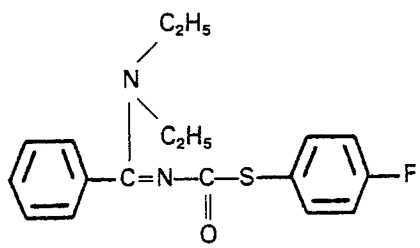 | $n_D^{26}$ 1.592 |
| 34 | 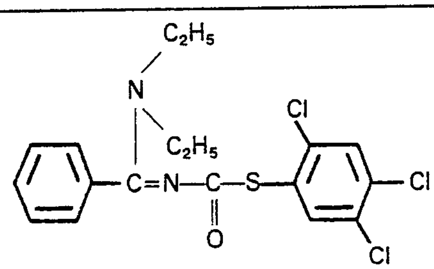 | m.p. 107 ~ 109°C |
| 35 | 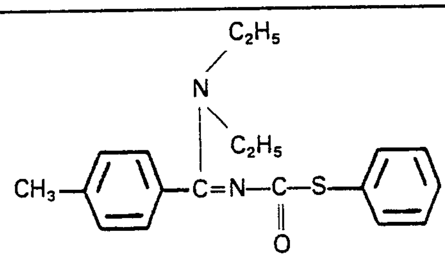 | m.p. 61 ~ 63°C |
| 36 | 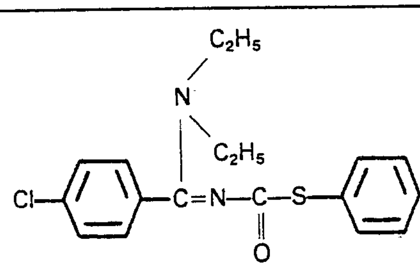 | m.p. 79 ~ 81°C |

TABLE 1 (continued)

| Compound No. | Structural formula | Physical property |
| --- | --- | --- |
| 37 | 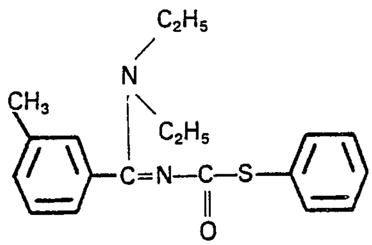 | m.p. 58 60°C |
| 38 | 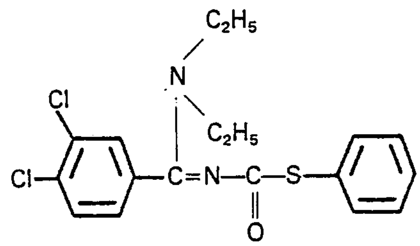 | m.p. 71 ~ 72°C |
| 39 | 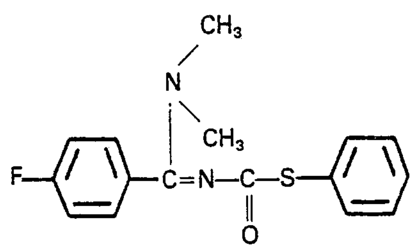 | m.p. 85 ~ 87°C |
| 40 | 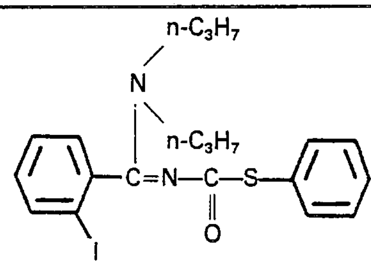 | $n_D^{29}$ 1.5945 |
| 41 | 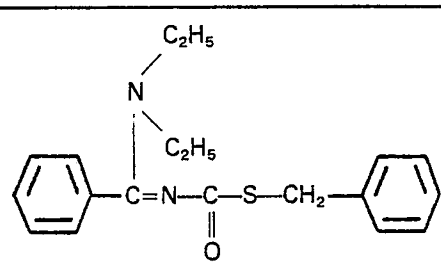 | m.p. 50 ~ 32°C |
| 42 | 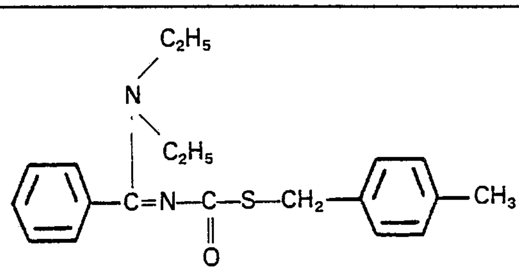 | m.p. 72 ~ 73°C |

TABLE 1 (continued)

| Compound No. | Structural formula | Physical property |
|---|---|---|
| 43 | 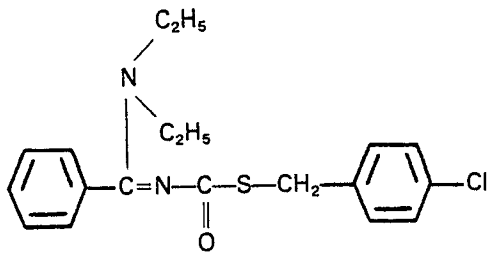 | m.p. 66 ~ 68°C |
| 44 | 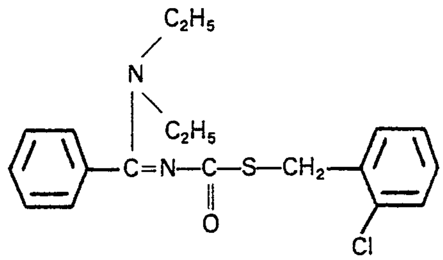 | $n_D^{25}$ 1.5880 |
| 45 | 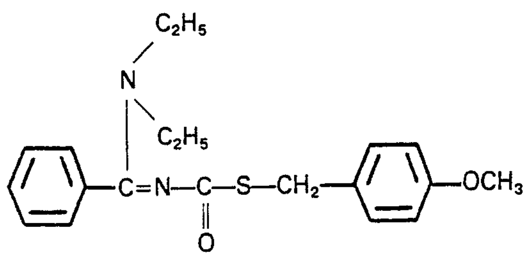 | $n_D^{28}$ 1.5820 |
| 46 | 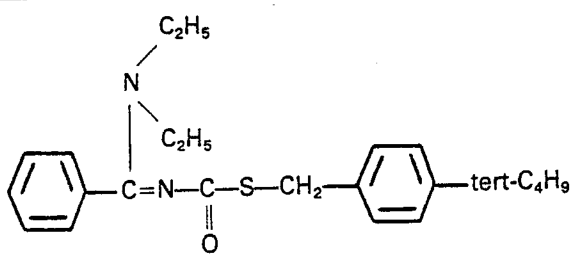 | $n_D^{29}$ 1.5815 |
| 47 | 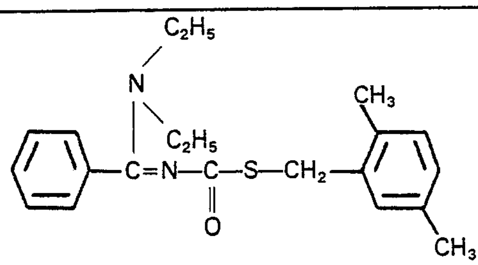 | $n_D^{25}$ 1.5975 |
| 48 | 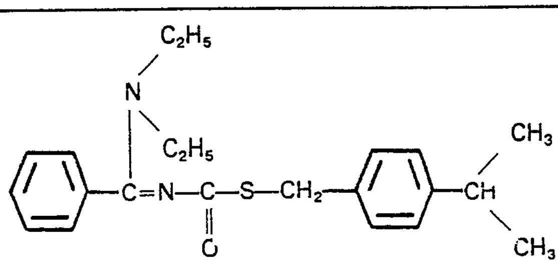 | $n_D^{27}$ 1.5742 |

TABLE 1 (continued)

| Compound No. | Structural Formula | Physical property |
|---|---|---|
| 49 | 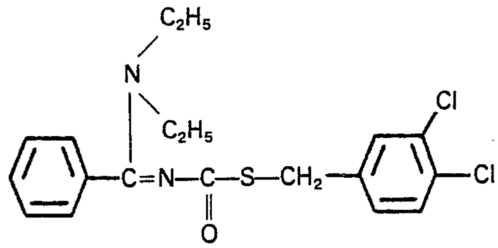 | $n_D^{25}$ 1.6015 |
| 50 | 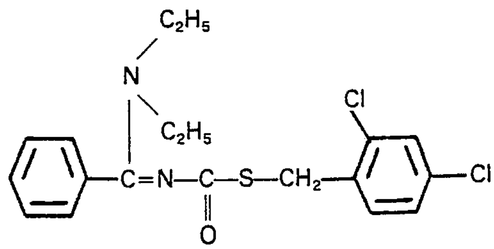 | $n_D^{25}$ 1.6077 |
| 51 | 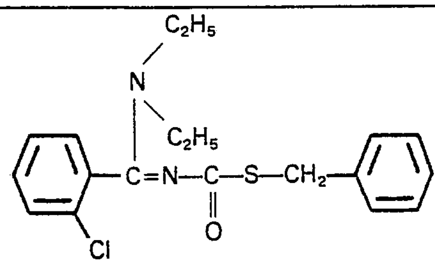 | $n_D^{26}$ 1.5921 |
| 52 | 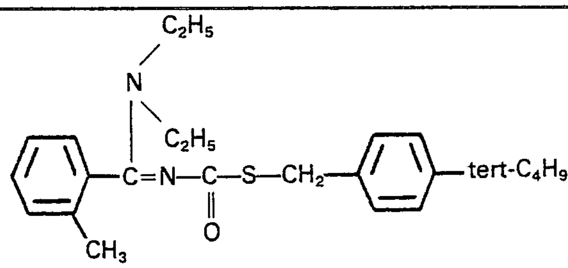 | $n_D^{29}$ 1.5793 |
| 53 | 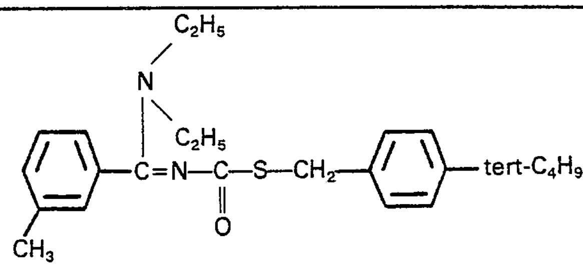 | $n_D^{29}$ 1.5762 |
| 54 | 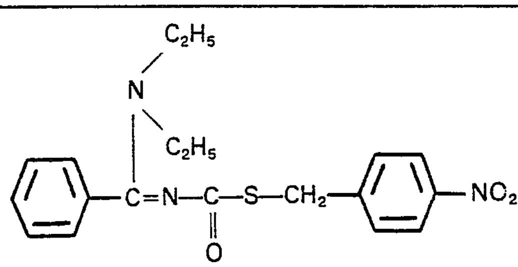 | TLC (benzene) Rf 0.15 |

TABLE 1 (continued)

| Compound No. | Structural Formula | Physical property |
|---|---|---|
| 55 | 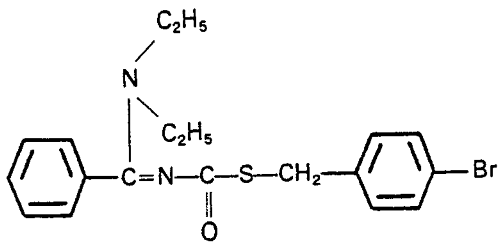 | m.p. 82 ~ 84°C |
| 56 | 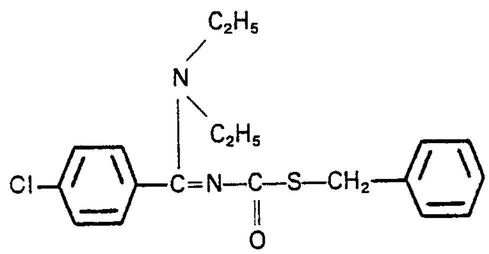 | m.p. 91 ~ 93°C |
| 57 | 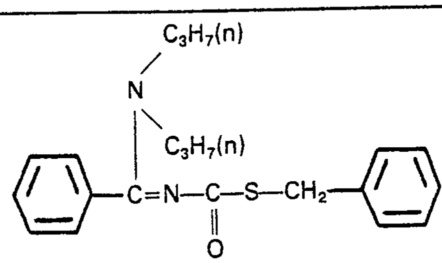 | $n_D^{22}$ 1.5778 |
| 58 | 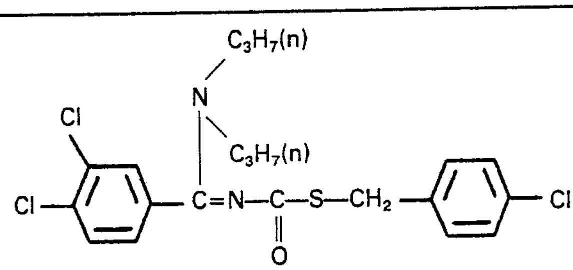 | $n_D^{27}$ 1.5858 |
| 59 | 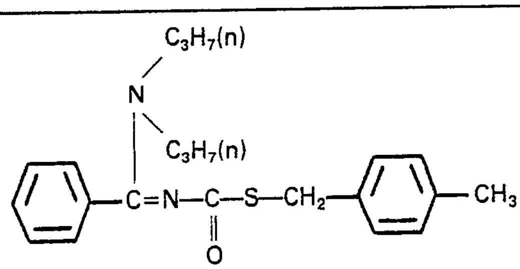 | $n_D^{27}$ 1.5814 |
| 60 | 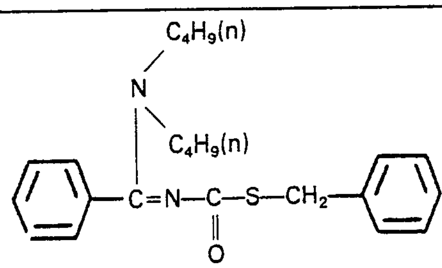 | $n_D^{20}$ 1.5662 |

TABLE 1 (continued)

| Compound No. | Structural formula | Physical property |
|---|---|---|
| 61 | 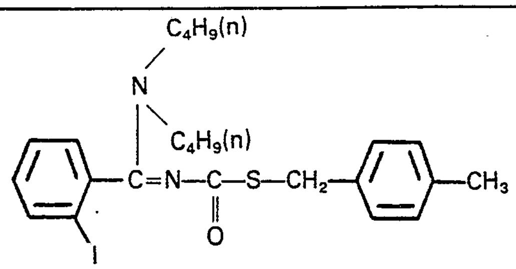 | $n_D^{21}$ 1.5603 |
| 62 | 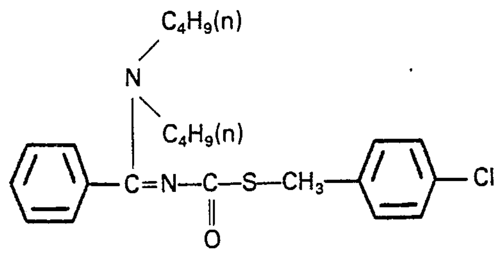 | $n_D^{21}$ 1.5603 |
| 63 | 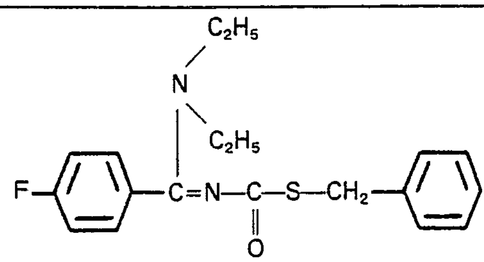 | m.p. 61 ~ 62°C |

The above listed benzamidine compounds Nos. 1 to 22 according to this invention can be prepared, for example, by a method described below.

As the first step, according to the following scheme, benzamidines and carbon disulfide are reacted with each other in a concentrated (conc.) aqueous sodium hydroxide solution to synthesize a sodium amidinodithioate:

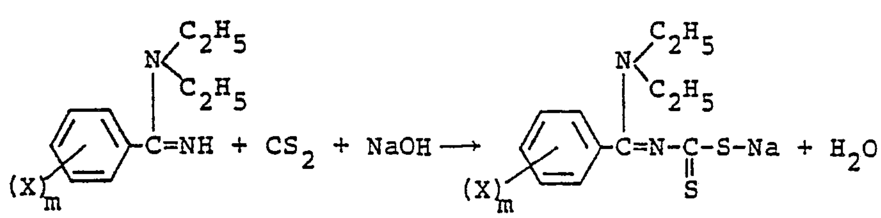

(wherein X and m have the same meanings as defined in the formula (II) or (I)).

The reaction can be carried out in water or a diluted (dil.) aqueous alcoholic solution at a temperature of −5 to 40°C.

After isolation of the sodium amidinothioate from the reaction system or without the isolation, the thus obtained dithioate is then reacted with a halide such as a lower alkyl halide, a lower alkenyl halide, a (halogenated) benzyl halide and the like; or with a salt of a metal such as nickel, cobalt and zinc with a mineral acid such as hydrochloric acid, sulfuric acid, nitric acid and the like, at a temperature of 0 to 50°C to synthesize various benzamidine compounds according to this invention. This reaction can be also carried out in water or a dil. aqueous alcoholic solution.

The above listed benzamidine compounds Nos. 23 to 40 of the present invention can be easily prepared according to the following scheme by reacting benzamidines with S-phenyl chlorothioformate:

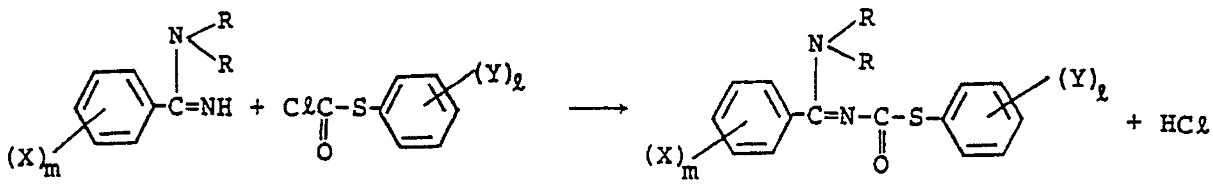

(wherein R, X, Y, m and l have the same meanings as defined in above formula (III)).

This reaction can be carried out at 0 to 60°C in an organic solvent such as ethyl ether, benzene, toluene, dichloroethane, chloroform and the like, in the presence of a dehydrochloric acid agent such as pyridine, a tertiary amine, e.g., triethylamine and dimethylaniline, sodium hydroxide, potassium carbonate and the like. S-phenyl chlorothioformate may be used in an amount of 0.5 to 5 moles per mole of benzamidines and the dehydrochloric acid agent may be preferably used in an amount of 0.5 to 10 moles per mole of benzamidines.

The above listed benzamidine compounds Nos. 41 to 63 of the present invention can be easily prepared according to the following scheme by reacting benzamidines with S-benzyl chlorothioformate:

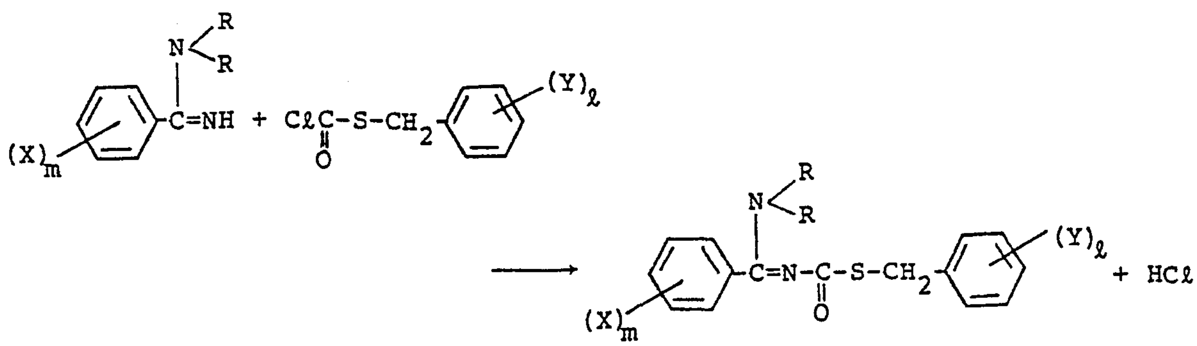

(wherein, R, X, Y, m and l have the same meanings as defined in above formula (IV)).

This reaction can be carried out at 0 to 60°C in an organic solvent such as ethyl ether, benzene, toluene, dichloroethane, chloroform and the like, in the presence of a dehydrochloric acid agent such as pyridine, a tertiary amine, e.g., triethylamine and dimethylaniline, sodium hydroxide, potassium carbonate and the like. S-benzyl chlorothioformate may be used in an amount of 0.5 to 5 moles per mole of benzamidines and the dehydrochloric acid agent may be preferably used in an amount of 0.5 to 10 moles per mole of benzamidines.

The synthesis of the compounds according to this invention will be explained in more detail by the following Synthesis.

## Synthesis 1

Synthesis of N,N-diethyl-N'-n-propyldithiocarboxybenzamidine (Compound No. 3)

To a mixture of 5.0 g (0.028 mol) of N,N-diethylbenzamidine and 2.3 g (0.030 mol) of carbon disulfide was added 50 ml of water, and then 1.3 g (0.032 mol) of sodium hydroxide dissolved in 5 ml of water was added dropwise thereto under ice cooling with vigorous stirring. During the addition, the reaction mixture gradually turned orange.

After stirring for further one hour, 3.5 g (0.024 mol) of n-propylbromide was added dropwise (the reaction mixture turned yellow) and stirring was continued for 30 minutes. The resultant reaction mixture was extracted with 60 ml of chloroform and the extract was neutralized with 2N hydrochloric acid, washed with water and dried over anhydrous sodium sulfate. Chloroform was removed from the resultant extract under reduced pressure to give an oily substance.

The oily substance was subjected to column chromatography (Wako-gel C-200) with use of benzene to isolate 4.3 g of the desired product having a refraction index of $n_d^{28}$ 1.6160 as an orange oily substance.

NMR spectrum (in deutero chloroform); δ value (ppm):

1.0 (t, 3H, $-SCH_2CH_2CH_3$)
1.1 ~ 1.5 (6H, $-N-(CH_2CH_3)_2$)
1.7 (m, 2H, $-SCH_2CH_2CH_3$)
3.1 (t, 2H, $-SCH_2CH_2CH_3$)
3.2 ~ 4.0 (4H, $-N-(CH_2CH_3)_2$)
7.5 (5H, 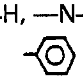).

Synthesis 2

Synthesis of N,N-diethyl-N'-benzyldithiocarboxybenzamidine (Compound No. 8)

To a mixture of 5 g (0.028 mol) of N,N-diethylbenzamidine and 2.3 g (0.030 mol) of carbon disulfide was added 40 ml of water, and 5 ml of aqueous solution containing 1.3 g (0.032 mol) of sodium hydroxide was then added dropwise slowly thereto under ice cooling with vigorous stirring.

After stirring for one hour, 4.9 g (0.028 mol) of benzylbromide was added dropwise and stirring was further continued for one hour and half. The resultant reaction mixture was extracted with 60 ml of chloroform and the extract was neutralized with 2N hydrochloric acid, washed with water and dried over anhydrous sodium sulfate. Chloroform was removed from the extract under reduced pressure to give an oily substance.

The oily substance was subjected to column chromatography (Wako-gel C-200) with use of benzene to isolate the desired product having a refraction index of $n_d^{27}$ 1.6540 as an orange oily substance.

NMR spectrum (in deutero chloroform); δ value (ppm):

1.0 ~ 1.6 (6H, $-N-(CH_2CH_3)_2$)

3.1 ~ 3.9 (4H, $-N-(CH_2CH_3)_2$)

4.4 (S, 2H, 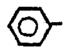 $-CH_2-$)

7.2 ∿ 7.7 (10H, 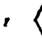 - x 2).

Synthesis 3

Synthesis of nickel bis(N,N-diethylbenzamidine-N'-carbodithioate) (Compound No. 10)

To an aqueous solution containing 2.1 g (0.05 mol) of sodium hydroxide dissolved in 3.2 ml of water was added 8.8 g (0.05 mol) of N,N-diethylbenzamidine, and 4.6 g (0.06 mol) of carbon disulfide was added dropwise thereto under room temperature with vigorous stirring, keeping the reaction temperature at 40°C or lower.

Crystals deposited after about 30 minutes were filtered with suction and the obtained crystal residue was washed twice with 30 ml portions of ethyl ether to give 11.8 g of sodium N,N-diethylbenzamidino-N'-dithioate as a yellow crystal.

After dissolving 11.8 g (0.043 mol) of the thus obtained crystal in 200 ml of water, was added 50 ml of an aqueous solution containing 5.6 g (0.022 mol) of nickel sulfate hexahydrate under room temperature with stirring. Deposited crystals were combined, washed with water and dried at 80°C under reduced pressure to give 9.3 g of the desired product having a decomposition temperature of 184 to 189°C as a common brick colored powdery crystal.

Elemental analysis (for $C_{12}H_{30}N_4S_4Ni$);

| | H(%) | C(%) | N(%) | S(%) | Ni(%) |
|---|---|---|---|---|---|
| Calculated: | 5.39 | 51.34 | 9.98 | 22.8 | 10.4 |
| Found: | 5.28 | 49.84 | 9.63 | 21.4 | 10.8 |

Synthesis 4

Synthesis of zinc bis(N,N-diethylbenzamidino-N'-carbodithioate) (Compound No. 11)

After dissolving 2.1 g (0.05 mol) of sodium hydroxide in 3.2 ml of water, 8.8 g (0.05 mol) of N,N-diethylbenzamidine was added thereto and then 4.6 g (0.06 mol) of carbon disulfide was further added dropwise under room temperature with vigorous stirring, keeping the reaction temperature at 40°C or lower.

Crystals deposited after 40 minutes were filtered with suction and washed twice with 30 ml portions of ethyl ether after removing water thoroughly to give 12.1 g of sodium N,N-diethylbenzamidino-N'-dithioate as a yellow crystal.

After dissolving 12.1 g (0.045 mol) of the thus obtained crystal in 200 ml of water, 50 ml of an aqueous solution containing 6.3 g (0.022 mol) of zinc sulfate heptahydrate was added dropwise thereto. Deposited crystals were combined, washed with water sufficiently and dried at 50°C under reduced pressure to give 9.6 g of the desired product having a melting point of 96 to 98°C as a yellow powdery crystal.

Elemental analysis (for $C_{24}H_{30}N_4S_4Zn$);

| | H(%) | C(%) | N(%) | S(%) | Zn(%) |
|---|---|---|---|---|---|
| Calculated: | 5.32 | 50.73 | 9.86 | 22.5 | 11.5 |
| Found: | 5.20 | 50.28 | 9.64 | 22.3 | 11.7 |

Synthesis 5

Synthesis of N,N-diethyl-N'-phenylthiocarboxybenzamidine (Compound No. 23)

To a mixture of 6 g (0.035 mol) of N,N-diethylbenzamidine and 4.0 g (0.039 mol) of triethylamine was added 150 ml of toluene, and 30 ml of a toluene solution containing 6.0 g (0.035 mol) of S-phenyl

chlorothioformate was then added dropwise slowly thereto under cooling. The resultant reaction mixture was stirred for 30 minutes, allowed to rise to room temperature and stirred further for one hour.

After completion of the reaction, cold water was added and the formed salt was dissolved and removed. The toluene phase of the thus obtained reaction mixture was subjected to treatment with 2N aqueous hydrochloric acid solution, followed by washing with water until the reaction mixture became neutral. After the toluene phase was dried over anhydrous magnesium sulfate, the solvent was removed to give a crude crystal. The thus obtained crude crystal was recrystallized from a mixture solvent of ethanol and water to give 7.2 g of the desired product having a melting point of 63 to 65°C as a colorless needle crystal.

Elemental analysis (for $C_{18}H_{20}ON_2S$);

| | H(%) | C(%) | N(%) |
|---|---|---|---|
| Calculated: | 6.45 | 69.19 | 8.97 |
| Found: | 6.48 | 69.28 | 8.98 |

Synthesis 6

Synthesis of N,N-diethyl-N'-(p-bromophenyl)thiocarboxybenzamidine (Compound No. 32)

To a mixture of 7.4 g (0.042 mol) of N,N-diethylbenzamidine and 4.7 g (0.046 mol) of triethylamine was added 180 ml of benzene, and 50 ml of a benzene solution containing 10.5 g (0.042 mol) of S-(p-bromophenyl)chlorothioformate was then added dropwise thereto under ice cooling. The resultant reaction mixture was stirred for 40 minutes and further stirred at room temperature for 2 hours to complete the reaction.

After dissolving and removing deposited salts with water, the mixture was treated with 2N hydrochloric acid. Then the benzene phase of the mixture was washed with water for neutralization, dried over anhydrous sodium sulfate and the solvent was removed to give a colorless crude crystal. The thus obtained crystal was recrystallized from a mixture solvent of ethyl ether and petroleum ether to give 10.0 g of the desired product having a melting point of 82 to 84°C as a colorless needle crystal.

Elemental analysis (for $C_{18}H_{19}ON_2SBr$);

| | H(%) | C(%) | N(%) |
|---|---|---|---|
| Calculated: | 4.89 | 55.26 | 7.16 |
| Found: | 4.87 | 55.01 | 7.18 |

Synthesis 7

Synthesis of N,N-diethyl-N'-(p-tert-butylphenyl)thiocarboxybenzamidine (Compound No. 31)

Into 200 ml of toluene was dissolved a mixture of 12.7 g (0.072 mol) of N,N-diethylbenzamidine and 8.1 g (0.079 mol) of triethylamine, and 50 ml of a toluene solution containing 16.4 g (0.072 mol) of S-(p-tert-butylphenyl)-chlorothioformate was then added dropwise thereto under cooling. The resultant reaction mixture was stirred for 45 minutes, allowed to rise to room temperature and further stirred for 1.5 hours.

Salts deposited with cold water were dissolved and removed, then the mixture was treated with 2N aqueous hydrochloric acid solution, followed by washing with water until the mixture become neutral. After the toluene phase of the mixture was dried over anhydrous magnesium sulfate, the solvent was removed on an evaporator to give an oily substance. The thus obtained oily substance was subjected to column chromatography (Wako-gel C-300) with use of a mixture solvent of 1:10 ethyl acetate-n-hexane (by volume ratio) to isolate 10.3 g of the desired product having a refraction index of $n_d^{18}$ 1.5885 as a yellow viscous oily substance.

Elemental analysis (for $C_{22}H_{28}ON_2S$);

| | H(%) | C(%) | N(%) |
|---|---|---|---|
| Calculated: | 7.66 | 71.70 | 7.60 |
| Found: | 7.58 | 71.26 | 7.17 |

Synthesis 8

Synthesis of N,N-diethyl-N'-benzylthiocarboxybenzamidine (Compound No. 41)

To a mixture of 8.1 g (0.046 mol) of N,N-diethylbenzamidine and 5.1 g (0.05 mol) of triethylamine was added 150 ml of toluene, and 10.0 g (0.046 mol) of S-benzyl chlorothioformate dissolved in 30 ml of toluene was then added dropwise slowly through a dropping funnel under cooling. During the addition, the reaction mixture was stirred vigorously. Stirring was continued for 3 hours after completion of addition and the reaction mixture was allowed to rise to room temperature and further stirred for one hour. After completion of reaction, deposited salts were filtered and removed, and the toluene phase of the filtrate was treated with 2N aqueous hydrochloric acid solution, followed by washing with water for neutralization. After the toluene phase was dried over anhydrous magnesium sulfate, the solvent was removed on an

evaporator to deposit a crystal. The thus obtained crystal was recrystallized from a mixture solvent of ethyl ether and petroleum benzine and allowed to stand in a refrigerator overnight to give 12.8 g of the desired product having a melting point of 50 to 52°C as a colorless prismatic crystal.

Elemental analysis (for $C_{19}H_{22}ON_2S$);

| | H(%) | C(%) | N(%) |
|---|---|---|---|
| Calculated: | 6.79 | 69.90 | 8.58 |
| Found: | 6.88 | 70.04 | 8.52 |

Synthesis 9

Synthesis of N,N-diethyl-N'-benzylthiocarboxy-o-chlorobenzamidine (Compound No. 51)

Into 200 ml of toluene was dissolved a mixture of 6.1 g (0.031 mol) of trichloromethyl chloroformate and 5.5 g (0.044 mol) of benzyl mercaptan, and 12.4 g (0.123 mol) of triethylamine was then added dropwise thereto under ice cooling over 20 minutes. The reaction mixture was stirred for 2 hours to synthesize S-benzyl chlorothioformate. To the resultant reaction mixture was added dropwise 11.2 g (0.053 mol) of N,N-diethyl-o-chlorobenzamidine, then the mixture was stirred for one hour and allowed to stand at room temperature overnight. After the organic phase of the mixture was washed with water, 2N hydrochloric acid and water in turn and dried over anhydrous magnesium sulfate, the solvent was removed to give an oily liquid. The thus obtained oily liquid was subjected to column chromatography (Wako-gel C-300) with use of a mixture solvent of 15:1 toluene-ethyl acetate (by volume ratio) to isolate 4.7 g of the desired product having a refraction index of $n_d^{16}$ 1.5921 as an orange oily substance.

Elemental analysis (for $C_{19}H_{21}ON_2SCl$);

| | H(%) | C(%) | N(%) |
|---|---|---|---|
| Calculated: | 5.87 | 63.23 | 7.76 |
| Found: | 6.54 | 62.98 | 7.42 |

When the benzamidine group compound according to this invention is to be applied as agricultural fungicides, the compound may be formulated for use to the preparations commonly employed as an agricultural fungicide, for example, dusts, granules, fine granules, wettable powders, emulsifiable concentrates, tablets, oily agents aerosol, fumigants and so on, with admixture of an inert solid carrier, inert liquid carrier or emulsifying dispersant.

As the inert carrier, there may be mentioned, for example, talc, clay, kaoline, diatomaceous earth, white carbon, calcium carbonate, potassium chlorate, saltpeter, wood flour, starch, nitrocellulose, gum arabic, vinyl chloride, carbon dioxide, Fleon, propane, butane and so on.

The agricultural fungicidal composition of this invention may also optionally be blended with any auxiliary agents for preparation, for example, spreaders, dispersing agents, emulsifying agents and the like as usually done in the art.

Moreover, the agricultural fungicidal composition of this invention may also be admixed with herbicides, insecticides, other agricultural chemicals, fertilizers, e.g., urea, ammonium sulfate, ammonium phosphate, potassic fertilizers, soil conditioners and the like.

The agricultural fungicidal compositions of this invention which may be formulated into various types of preparations as above, may be applied, for example, in a paddy or upland field at 1—5000 g, preferably 10—1000 g of the active ingredient per 10 ares for pre- or post-emergency foliage spraying or soil drenching or spraying onto water to control diseases effectively.

The fungicidal composition according to this invention shows excellent effect particularly upon powdery mildew of various field crops even in smaller amounts as compared with commercially available fungicidal compositions. In addition, the fungicidal composition according to this invention also has excellent effect upon such plant diseases as gray mold, anthracnose, blast disease, brown spot, bacterial soft rot, bacterial leaf blight, screlotium disease and canker (*Valsa mali*). Furthermore, the fungicidal composition according to this invention shows extremely low phytotoxicity against field crops as well as extremely low toxicity against man and beast.

Examples of the preparation of the present agricultural fungicidal composition are given below. All parts are given by weight hereinafter unless otherwise stated.

Example 1

Emulsifiable concentrates

(1) Twenty parts of N,N-diethyl-N'-methyldithiocarboxybenzamidine (Compound No. 1), 5 parts of Toxanone (trade name of surfactant from Sanyo Kasei Kogyo K.K.) and 75 parts of xylene were homogeneously blended to give 100 parts of emulsifiable concentrates.

(2) Twenty parts of N,N-diethyl-N'-(p-methylphenyl)thiocarboxybenzamidine (Compound No. 24), 5 parts of Toxanone (trade name of surfactant from Sanyo Kasei Kogyo K.K.) and 75 parts of xylene were homogeneously blended to give 100 parts of emulsifiable concentrates.

(3) Twenty parts of N,N-diethyl-N'-benzylthiocarboxybenzamidine (Compound No. 41), 5 parts of Toxanone (trade name of surfactant from Sanyo Kasei Kogyo K.K.) and 75 parts of xylene were homogeneously blended to give 100 parts of emulsifiable concentrates.

Example 2

Wettable powders

(1) Twenty parts of N,N-diethyl-N'-benzyldithiocarboxybenzamidine (Compound No. 8), one part of Demol EP powder (trade name of surfactant from Kao-Atlas Co., Ltd.), 20 parts of white carbon and 59 parts of the talc were homogeneously blended and pulverized to give 100 parts of wettable powders.

(2) Twenty parts of N,N-diethyl-N'-(o-methylphenyl)-thiocarboxybenzamidine (Compound No. 27), one part of Demol EP powder (trade name of surfactant from Kao-Atlas Co., Ltd.), 20 parts of white carbon and 59 parts of talc were homogeneously blended and pulverized to give 100 parts of wettable powders.

(3) Twenty parts of N,N-diethyl-N'-benzylthiocarboxy-o-chlorobenzamidine (Compound No. 51), one part of Demol EP powder (trade name of surfactant from Kao-Atlas Co., Ltd.), 20 parts of white carbon and 59 parts of talc were homogeneously blended and pulverized to give 100 parts of wettable powders.

Example 3

Dusts

(1) Two parts of zinc bis (N,N-diethyl-p-methylbenzamidino-N'-carbodithioate) (Compound No. 18) and 98 parts of talc were homogeneously blended to give 100 parts of dusts.

(2) Two parts of N,N-diethyl-N'-(p-chlorophenyl)thiocarboxybenzamidine (Compound No. 29) and 98 parts of talc were homogeneously blended to give 100 parts of dusts.

(3) Two parts of N,N-diethyl-N'-(p-nitrobenzyl)thiocarboxybenzamidine (Compound 54) and 98 parts of talc were homogeneously blended to give 100 parts of dusts.

Example 4

Granules or tablets

(1) Ten parts of zinc bis (N,N-diethyl-p-chlorobenzamidino-N'-carbodithioate) (Compound No. 22), 15 parts of starch, 72 parts of bentonite and 3 parts of sodium salt of lauryl alcohol sulfate were homogeneously blended and pulverized to give 100 parts of granules or tablets.

(2) Ten parts of N,N-diethyl-N'-phenylthiocarboxy-p-chlorobenzamidine (Compound No. 36), 15 parts of starch, 72 parts of bentonite and 3 parts of sodium salt of lauryl alcohol sulfate were homogeneously blended and pulverized to give 100 parts of granules or tablets.

(3) Ten parts of N,N-di-n-butyl-N'-benzylthiocarboxybenzamidine (Compound No. 60), 15 parts of starch, 2 parts of bentonite and 3 parts of sodium salt of lauryl alcohol sulfate were homogeneously blended and pulverized to give 100 parts of granules or tablets.

Next, the effect of the agricultural fungicidal composition according to this invention will be explained more concretely by way of the following Experiments.

The numbers of the test compounds correspond to those exemplified in the above table.

Experiment 1

Effect on powdery mildew in cucumber

Cucumber seedlings (variety: Sagamihanjiro-fushinari), planted one per 6 cm diameter plastic pot, were used as host plants at the stage of 15 days after sowing. Each host plant was treated with a diluted emulsifiable concentrate or wettable powder prepared in the same manner as in Example 1 or 2, respectively, and containing 1000 ppm of each of active compounds by spraying at a rate of 20 ml per pot.

After air-drying each host plant was inoculated with *Sphaerotheca fuliginea* by spraying its spore suspension uniformly. The spore suspension had been previously prepared by brushing already infected cucumber leaves with a soft brush and letting the microorganism fall onto a petri dish, and diluting the conidia with distilled water to such a concentration that 10 conidia had been observed within the range of an optical microscope at 150 magnifications. The inoculated plants were kept in an air-conditioned and isolated glass greenhouse. Upon the emergence of the disease after about 10 days, the number of diseased spots on the first leaf was measured.

The percent efficacy of each of active compounds was calculated according to the following equation. The results are shown in Table 2.

$$\text{Percent Efficacy} = \left(1 - \frac{\text{Average number of diseased spots in treated groups}}{\text{Average number of diseased spots in untreated groups}}\right) \times 100$$

TABLE 2

| Test Compound No. | Efficacy (%) | Phytotoxicity |
|---|---|---|
| 1 | 100 | none |
| 2 | " | " |
| 3 | " | " |
| 4 | " | " |
| 5 | " | " |
| 6 | " | " |
| 7 | " | " |
| 8 | " | " |
| 9 | " | " |
| 10 | " | " |
| 11 | " | " |
| 12 | " | " |
| 13 | " | " |
| 14 | " | " |
| 15 | " | " |
| 16 | " | " |
| 17 | " | " |
| 18 | " | " |
| 19 | " | " |
| 20 | " | " |
| 21 | " | " |
| 22 | " | " |
| 23 | " | " |
| 24 | " | " |
| 25 | " | " |
| 26 | " | " |
| 27 | " | " |
| 28 | " | " |
| 29 | " | " |

TABLE 2 (cont'd)

| Test Compound No. | Efficacy (%) | Phytotoxicity |
|---|---|---|
| 30 | 100 | none |
| 31 | " | " |
| 32 | " | " |
| 33 | " | " |
| 34 | " | " |
| 35 | " | " |
| 36 | " | " |
| 37 | " | " |
| 38 | " | " |
| 39 | " | " |
| 40 | " | " |
| 41 | " | " |
| 42 | " | " |
| 43 | " | " |
| 44 | " | " |
| 45 | " | " |
| 46 | " | " |
| 47 | " | " |
| 48 | " | " |
| 49 | " | " |
| 50 | " | " |
| 51 | " | " |
| 52 | " | " |
| 53 | " | " |
| 54 | " | " |
| 55 | " | " |
| 56 | " | " |
| 57 | 58 | " |
| 58 | 70 | " |
| 59 | 60 | " |

TABLE 2 (cont'd)

| Test Compound No. | Efficacy (%) | Phytotoxicity |
|---|---|---|
| 60 | 42 | none |
| 61 | 40 | " |
| 62 | 40 | " |
| 63 | 100 | |

Experiment 2

Effect on powdery mildew in cucumber at low concentrations

Experiments were conducted according to the method in Experiment 1, except that 20 ml of each of diluted emulsifiable concentrates or wettable powders prepared in the same manner as in Example 1 or 2, respectively, and containing 200 ppm, 100 ppm or 50 ppm of each of active compounds was sprayed per groups.

The percent efficacy was calculated according to the same manner as in Experiment 1. The results are shown in Table 3.

TABLE 3

| Test Compound No. | Efficacy (%) | | |
|---|---|---|---|
| | Concentration of Active Ingredient | Concentration of Active Ingredient | Concentration of Active Ingredient |
| | 200 ppm | 100 ppm | 50 ppm |
| 1 | 90 | 81 | 60 |
| 2 | 100 | 93 | 82 |
| 3 | 96 | 86 | 65 |
| 4 | 100 | 89 | 65 |
| 5 | 100 | 88 | 60 |
| 6 | 92 | 88 | 70 |
| 7 | 92 | 86 | 65 |
| 8 | 94 | 84 | 46 |
| 9 | 100 | 88 | 67 |
| 10 | 100 | 100 | 91 |
| 11 | 100 | 100 | 92 |
| 12 | 100 | 98 | 89 |
| 13 | 100 | 96 | 78 |
| 14 | 100 | 100 | 99 |
| 15 | 100 | 97 | 88 |
| 16 | 100 | 100 | 84 |

TABLE 3 (cont'd)

| Test Compound No. | Efficacy (%) | | |
|---|---|---|---|
| | Concentration of Active Ingredient | Concentration of Active Ingredient | Concentration of Active Ingredient |
| | 200 ppm | 100 ppm | 50 ppm |
| 17 | 100 | 100 | 87 |
| 18 | 100 | 100 | 61 |
| 19 | 100 | 100 | 84 |
| 20 | 100 | 98 | 83 |
| 21 | 100 | 100 | 83 |
| 22 | 100 | 92 | 68 |
| 23 | 100 | 100 | 99 |
| 24 | " | 96 | 84 |
| 25 | " | 94 | " |
| 26 | " | 100 | 93 |
| 27 | " | " | 97 |
| 28 | " | " | 100 |
| 29 | " | 98 | 87 |
| 30 | " | 100 | 95 |
| 31 | " | 98 | 86 |
| 32 | " | 100 | 99 |
| 33 | " | " | 100 |
| 34 | " | " | 99 |
| 35 | " | " | 96 |
| 36 | " | 95 | 87 |
| 37 | " | 100 | 100 |
| 38 | " | " | " |
| 39 | 94 | 78 | 52 |
| 40 | 86 | 60 | 41 |
| 41 | 100 | 100 | 98 |
| 42 | " | " | 100 |
| 43 | " | " | " |

TABLE 3 (cont'd)

| Test Compound No. | Efficacy (%) | | |
|---|---|---|---|
| | Concentration of Active Ingredient | Concentration of Active Ingredient | Concentration of Active Ingredient |
| | 200 ppm | 100 ppm | 50 ppm |
| 44 | " | " | " |
| 45 | " | " | 98 |
| 46 | " | " | 83 |
| 47 | " | 98 | 79 |
| 48 | " | 80 | 70 |
| 49 | " | 100 | 100 |
| 50 | " | 96 | 72 |
| 51 | " | 100 | 100 |
| 52 | " | 98 | 80 |
| 53 | " | 96 | 70 |
| 54 | " | 71 | 52 |
| 55 | " | 78 | 62 |
| 56 | " | 100 | 92 |
| 63 | " | " | 98 |
| Control* | 86 | 41 | 23 |

*: N,N-diethyl-N'-ethylthiocarboxybenzamidine

## Experiment 3

Effect on canker in apple

Apple tree (variety: Fuji) was used as host plants. Branches at the stage of one year after germination having a length of 7 cm which had been cut from the apple tree were immersed for 10 minutes in a diluted emulsifiable concentrate or wettable powder prepared in the same manner as in Example 1 or 2, respectively, and containing 1000 ppm of each of active compounds.

After air-drying, the branch bark having a diameter off 5 mm was punched at the central portion thereof with a heated cork borer and the thus punched portion of the branch was inoculated with a spore suspension of *Valsa mali* which had been previously cultivated on a PDA medium (spore concentration: 5 $10^6$/ml). After inoculation, the host plants were kept at a temperature off 25°C and a relative humidity of 70% for 10 days. The number of diseased spots emerged was calculated and the percent efficacy of each of active compounds was calculated according to the same manner as in Experiment 1. The results are shown in Table 4.

TABLE 4

| Test Compound No. | Efficacy (%) | Phytotoxicity |
|---|---|---|
| 1 | 52 | none |
| 7 | 60 | " |
| 8 | 64 | " |
| 9 | 60 | " |
| 10 | 58 | " |
| 11 | 50 | " |
| 12 | 52 | " |
| 41 | 56 | " |
| 42 | 61 | " |
| 43 | 52 | " |
| 45 | 70 | " |
| 51 | 56 | " |
| 54 | 64 | " |
| Control* | 50 | " |

*: Topsin M; 1,2-bis(3-methoxycarbonyl-2-thioureido)benzene.

Experiment 4

Effect on powdery mildew in Tobacco

Tobacco seedlings (variety: MC-1), planted one per 12 cm diameter unglazed pot, were used as host plants at the stage of 4 to 5 leaves. Each host plant was treated with a diluted emulsifiable concentrates or wettable powders prepared in the same manner as in Example 1 or 2, respectively, and containing 500 ppm of each of active compounds by spraying at a rate of 500 ml per pot. After air-drying, each host plant was inoculated with *Erysiphe cichoracearum* by spraying its spore suspension uniformly. The spore suspension had been previously prepared in the same manner as in Experiment 1.

The inoculated plants were kept at 25°C in an air-conditioned and isolated greenhouse.

Upon the emergence of the disease after about 14 days, the number of diseased spots on the leaves was counted and the percent efficacy was calculated according to the same manner as in Experiment 1. The results are shown in Table 5.

TABLE 5

| Test Compound No. | Efficacy (%) | Phytotoxicity |
|---|---|---|
| 1 | 100 | none |
| 2 | 100 | " |
| 3 | 98 | " |
| 4 | 100 | " |
| 5 | 100 | " |
| 6 | 100 | " |
| 7 | 100 | " |
| 8 | 100 | " |
| 9 | 96 | " |
| 10 | 100 | " |
| 11 | 100 | " |
| 12 | 100 | " |
| 13 | 100 | " |
| 14 | 100 | " |
| 15 | 100 | " |
| 16 | 100 | " |
| 17 | 98 | " |
| 18 | 95 | " |
| 19 | 100 | " |
| 20 | 100 | " |
| 21 | 100 | " |
| 22 | 100 | " |
| 23 | 100 | " |
| 24 | " | " |
| 25 | " | " |
| 26 | " | " |
| 27 | " | " |
| 28 | " | " |
| 29 | " | " |
| 30 | " | " |

TABLE 5 (cont'd)

| Test Compound No. | Efficacy (%) | Phytotoxicity |
|---|---|---|
| 31 | 100 | none |
| 32 | " | " |
| 33 | " | " |
| 34 | " | " |
| 35 | " | " |
| 36 | " | " |
| 37 | " | " |
| 38 | " | " |
| 39 | 90 | " |
| 40 | 88 | " |
| 41 | 100 | " |
| 42 | " | " |
| 43 | " | " |
| 44 | " | " |
| 45 | " | " |
| 46 | " | " |
| 47 | " | " |
| 48 | " | " |
| 49 | " | " |
| 50 | " | " |
| 51 | " | " |
| 52 | " | " |
| 53 | " | " |
| 54 | " | " |
| 55 | " | " |
| 56 | " | " |
| 57 | 70 | " |
| 58 | 68 | " |
| 59 | 80 | " |
| 60 | 65 | " |
| 61 | 90 | " |
| 62 | 50 | " |
| 63 | 100 | " |

Experiment 5

Effect on sheath blight in rice plant

Rice seeds (variety: Nihonbare) were sown, 10 to the pot, in a series of 6 cm diameter plastic pots and grown to the 4 to 5 leaf stage. A diluted emulsifiable concentrate or wettable powder prepared in the same manner as in Example 1 or 2, respectively, and containing 500 ppm of each of active compounds by spraying at a rate of 20 ml per pot. After air-drying, each host plant was inoculated with the inoculum. The inoculum had been previously prepared by cultivating *Pellicularia sasaki* on a ban medium for about 14 days, inoculating the inoculum diluted with unhulled rice at the portion adjacent the ground and kept in a conditioned inoculation chamber (a temperature of 25°C and a relative humidity of 80%) to emerge the disease.

Seven to ten days after inoculation, diseased spots emerged on leaves was measured to calculate the number of leaves ($n_1$—$n_7$) at various degress of disease according to the following evaluation standard.

| | |
|---|---|
| 0: no infection observed | (number of leaves: $n_1$) |
| 1: slight infection observed | (number of leaves: $n_2$) |
| 2: diseased spots of less than 1 cm observed | (number of leaves: $n_3$) |
| 3: diseased spots of less than 2 cm observed | (number of leaves: $n_4$) |
| 4: diseased spots of less than 5 cm observed | (number of leaves: $n_5$) |
| 5: diseased spots of less than 10 cm observed | (number of leaves: $n_6$) |
| 6: diseased spots of more than 10 cm observed | (number of leaves: $n_7$) |

The percent efficacy of each of active compounds was calculated, based on the above observation, according to the following equation. The results are shown in Table 6.

$$\text{Percent Efficacy} = \left(1 - \frac{\text{treated groups } (0 \times n_1 + 1 \times n_2 + 2 \times n_3 + 3 \times n_4 + 4 \times n_5 + 5 \times n_6 + 6 \times n_7}{\text{untreated groups } (0 \times n_1 + 1 \times n_2 + 2 \times n_3 + 3 \times n_4 + 4 \times n_5 + 5 \times n_6 + 6 \times n_7}\right) \times 100$$

TABLE 6

| Test Compound No. | Efficacy (%) | Phytotoxicity |
|---|---|---|
| 42 | 90 | none |
| 45 | 87 | " |
| 51 | 100 | " |
| 53 | 95 | " |
| 54 | 89 | " |
| 56 | 90 | " |

Experiment 6

Effect on brown spot in rice plant (*Helminthosporium leaf spot*)

Rice seeds (variety: Nihonbare) were sown, 10 to the pot, in 6 cm diameter plastic pots and grown to the 4 to 5 leaf stage. Each host plants was treated with a diluted emulsifiable concentrate or wettable powder each prepared in the same manner as in Example 1 or 2, respectively, and containing 500 ppm of an active compound, at a rate of 20 ml per pot. After air-drying, the host plants were inoculated with *Cochliobolus miyabeanus* which had been cultivated in a rice straw extract medium at 28°C for 10 days and adjusted to such a concentration that 10—15 conidia had been observed within the range of an optical microscope at 150 magnifications. The inoculated plants were kept at 27°C, more than 95% relative humidity in an inoculation chamber for two days.

The number of diseased spots appeared on the upper two leaves was measured and the efficacy of each of active compounds was calculated according to the similar method as in Experiment 1. The results are shown in Table 7.

TABLE 7

| Test Compound No. | Efficacy (%) | Phytotoxicity |
|---|---|---|
| 41 | 75 | none |
| 43 | 89 | " |
| 45 | 80 | " |
| 47 | 92 | " |
| 49 | 87 | " |
| 51 | 98 | " |
| 60 | 76 | " |

**Claims**

1. A benzamidine group compound represented by the general formula (I):

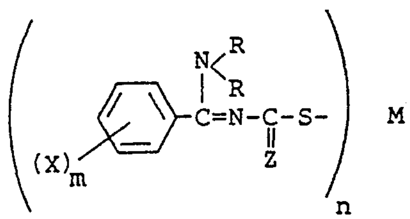

(I)

wherein R represents a $C_1$ to $C_4$ alkyl group; X represents a halogen atom or a $C_1$ to $C_4$ alkyl group; m represents an integer of 0, 1 or 2; Z represents an oxygen atom or a sulfur atoms; n represents an integer of 1 or 2; when n is 1, M represents a $C_1$ to $C_4$ alkyl group, a $C_3$ to $C_5$ alkenyl group, a phenyl group of the formula

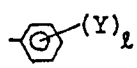

or a benzyl group of the formula

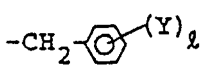

(in which Y represents a $C_1$ to $C_4$ alkyl group, a halogen atom, a $C_1$ to $C_4$ alkoxy group or a nitro group and l represents an integer of 0, 1, 2 or 3); and when n is 2, M represents a metal selected from nickel, cobalt and zinc; with the proviso that when Z is oxygen, M is not a $C_1$ to $C_4$ alkyl group.

2. The benzamidine group compound according to Claim 1, wherein said compound is represented by the general formula (II):

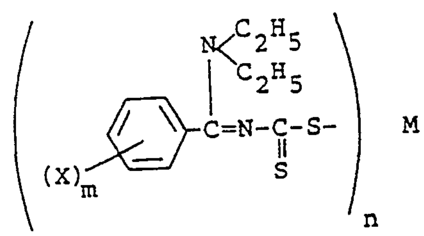

(II)

wherein X, m and n have the same meanings as defined in Claim 1, and when n is 1, M represents a $C_1$ to $C_4$ alkyl group, a $C_3$ to $C_5$ alkenyl group, a benzyl group or a halogenated benzyl group, and when n is 2, M has the same meaning as defined in Claim 1.

3. The benzamidine group compound according to Claim 2, wherein said benzamidine group compound is selected from the group consisting of (1) N,N-diethyl-N'-n-propyldithiocarboxybenzamidine, (2) N,N-diethyl-N'-benzyldithiocarboxybenzamidine, (3) Nickel bis(N,N-diethylbenzamidino-N'-carbodithioate), (4) Zinc bis(N,N-diethylbenzamidino-N'-carbodithioate) and (5) Cobalt bis(N,N-diethylbenzamidino-N'-carbodithioate).

4. The benzamidine group compound according to Claim 1, wherein said compound is represented by the general formula (III):

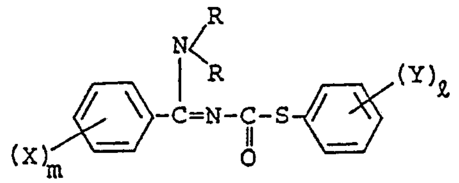

(III)

wherein R, X, m and l have the same meanings as defined in Claim 1, and Y represents a $C_1$ to $C_4$ alkyl group or a halogen atom.

5. The benzamidine group compound according to Claim 4, wherein said benzamidine group compound is selected from the group consisting of (1) N,N-diethyl-N'-phenylthiocarboxybenzamidine, (2) N,N-diethyl-N'-(p-bromophenyl)thiocarboxybenzamidine and (3) N,N-diethyl-N'-(p-tertbutylphenyl)thiocarboxybenzamidine.

6. The benzamidine group compound according to Claim 1, wherein said compound is represented by the general formula (IV):

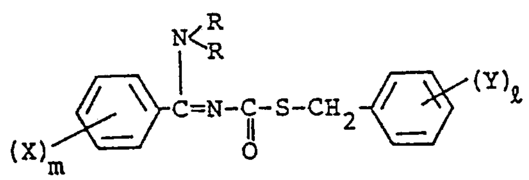

(IV)

wherein R, X, m and Y have the same meanings as defined in Claim 1, and l represents an integer of 0, 1 or 2.

7. The benzamidine group compound according to Claim 6, wherein said benzamidine group compound is selected from the group consisting of (1) N,N-diethyl-N'-benzylthiocarboxybenzamidine and (2) N,N-diethyl-N'-benzylthiocarboxy-o-chlorobenzamidine.

8. An agricultural fungicidal composition which comprises as an active ingredient, a benzamidine group compound represented by the general formual (I) of Claim 1.

9. The agricultural fungicidal composition according to Claim 8, wherein said compound is represented by the general formula (II) of Claim 2.

10. The agricultural fungicidal composition according to Claim 9, wherein said benzamidine group compound is selected from the group consisting of (1) N,N-diethyl-N'-n-propyldithiocarboxybenzamidine, (2) N,N-diethyl-N'-benzyldithiocarboxybenzamidine, (3) Nickel bis(N,N-diethylbenzamidino-N'-carbodithioate), (4) Zinc bis(N,N-diethylbenzamidino-N'-carbodithioate) and (5) Cobalt bis(N,N-diethylbenzamidino-N'-carbodithioate).

11. The agricultural fungicidal composition according to Claim 8, wherein said compound is represented by the general formula (III) of Claim 4.

12. The agricultural fungicidal composition according to Claim 11, wherein said benzamidine group compound is selected from the group consisting of (1) N,N-diethyl-N'-phenylthiocarboxybenzamidine, (2) N,N-diethyl-N'-(p-bromophenyl)thiocarboxybenzamidine and (3) N,N-diethyl-N'-(p-tert-butylphenyl)thiocarboxybenzamidine.

13. The agricultural fungicidal composition according to Claim 8, wherein said compound is represented by the general formula (IV) of Claim 6.

14. The agricultural fungicidal composition according to Claim 13, wherein said benzamidine group compound is selected from the group consisting of (1) N,N-diethyl-N'-benzylthiocarboxybenzamidine and (2) N,N-diethyl-N'-benzylthiocarboxy-o-chlorobenzamidine.

**Patentansprüche**

1. Eine eine Benzamidingruppe enthaltende Verbindung der allgemeinen Formel (I)

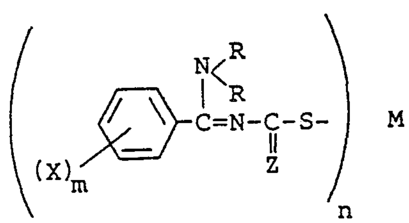

(I)

worin R eine $C_{1-4}$-Alkylgruppe bedeutet; X ein Halogenatom oder eine $C_{1-4}$-Alkylgruppe bedeutet; m die Zahl 0, 1 oder 2 bedeutet; Z ein Sauerstoffatom oder ein Schwefelatom ist; n die Zahl 1 oder 2 ist; wobei

dann, wenn n 1 ist, M eine $C_{1-4}$-Alkylgruppe, eine $C_{3-5}$-Alkenylgruppe, eine Phenylgruppe der Formel

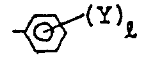

oder eine Benzylgruppe der Formel

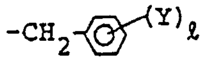

(in welcher Y eine $C_{1-4}$-Alkoxygruppe, ein Halogenatom, eine $C_{1-4}$-Alkoxygruppe oder eine Nitrogruppe bedeutet und l die Zahl 0, 1, 2 oder 3 bedeutet) bedeutet und wenn n 2 ist, M ein Metall, ausgewählt aus Nickel, Kobalt und Zink bedeutet; unter der Voraussetzung, dass dann, wenn Z Sauerstoff ist, M keine $C_{1-4}$-Alkylgruppe ist.

2. Eine eine Benzamidingruppe enthaltende Verbindung gemäss Anspruch 1, worin die Verbindung die allgemeine Formel (II)

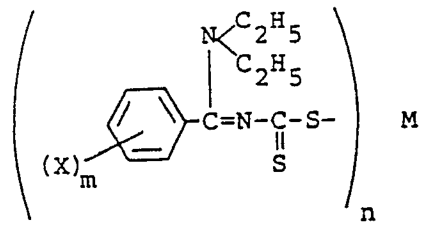

(II)

hat, worin X, m und n die in Anspruch 1 angegebenen Bedeutungen haben und, wenn n 1 ist, M eine $C_{1-4}$-Alkylgruppe, eine $C_{3-5}$-Alkenylgruppe, eine Benzylgruppe oder eine halogenierte Benzylgruppe bedeutet, und wenn n 2 ist, M die gleiche Bedeutung wie in Anspruch 1 hat.

3. Eine eine Benzamidingruppe enthaltende Verbindung gemäss Anspruch 2, in welcher die Benzamidingruppen enthaltende Verbindung ausgewählt ist aus der Gruppe, bestehend aus (1) N,N-Diethyl-N'-n-propyldithiocarboxybenzamidin, (2) N,N-Diethyl-N'-benzyldithiocarboxybenzamidin, (3) Nickel- bis(N,N-diethylbenzamidino-N'-carbodithioat), (4) Zink-bis(N,N-diethylbenzamidino-N'-carbodithioat) und (5) Kobalt-bis(N,N-diethylbenzamidino-N'-carbodithioat).

4. Eine eine Benzamidingruppe enthaltende Verbindung gemäss Anspruch 1, in welcher die Verbindung die allgemeine Formel (III)

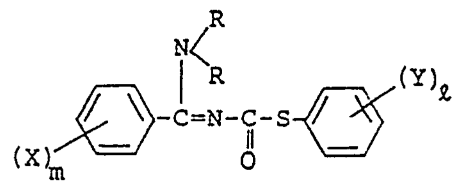

(III)

hat, in welcher R, X, m und l die in Anspruch 1 angegebenen Bedeutungen haben und Y eine $C_{1-4}$-Alkylgruppe oder ein Halogenatom bedeutet.

5. Eine eine Benzamidingruppe enthaltende Verbindung gemäss Anspruch 4, wobei die Benzamidingruppe enthaltende Verbindung ausgewählt ist aus der Gruppe, bestehend aus (1) N,N-Diethyl-N'-phenylthiocarboxybenzamidin, (2) N,N-Diethyl-N'-(p-bromophenyl)thiocarboxybenzamidin und (3) N,N-Diethyl-N'-(p-tert-butylphenyl)thiocarboxybenzamidin.

6. Eine eine Benzamidingruppe enthaltende Verbindung gemäss Anspruch 1, wobei die Verbindung die allgemeine Formel (IV)

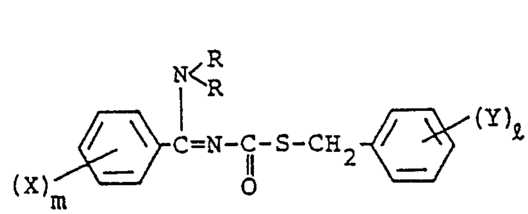

(IV)

hat, in welcher R, X, m und Y die in Anspruch 1 angegebenen Bedeutungen haben und l die Zahl 0, 1 oder 2 bedeutet.

7. Eine eine Benzamidingruppe enthaltende Verbindung gemäss Anspruch 6, in welcher die Benzamidingruppe enthaltende Verbindung ausgewählt ist aus der Gruppe bestehend aus (1) N,N-Diethyl-N'-benzylthiocarboxybenzamidin und (2) N,N-Diethyl-N'-benzylthiocarboxy-o-chlorobenzamidin.

8. Eine landwirtschaftliche Fungizidzusammensetzung, umfassend als aktiven Bestandteil eine Benzamidingruppen enthaltende Verbindung der allgemeinen Formel (I) gemäss Anspruch 1.

9. Eine landwirtschaftliche Fungizidzusammensetzung gemäss Anspruch 8, worin die Verbindung die Verbindung der allgemeinen Formel (II) gemäss Anspruch 2 ist.

10. Eine landwirtschaftliche Fungizidzusammensetzung gemäss Anspruch 9, in welcher die Benzamidingruppen enthaltende Verbindung ausgewählt ist aus der Gruppe, bestehend aus (1) N,N-Diethyl-N'-n-propyldithiocarboxybenzamidin, (2) N,N-Diethyl-N'-benzyldithiocarboxybenzamidin, (3) Nickel-bis(N,N-diethylbenzamidino-N'-carbodithioat), (4) Zink-bis(N,N-diethylbenzamidino-N'-carbodithioat) und (5) Kobalt-bis(N,N-diethylbenzamidino-N'-carbodithioat).

11. Eine landwirtschaftliche Fungizidzusammensetzung gemäss Anspruch 8, in welcher die Verbindung die Verbindung der allgemeinen Formel (II) gemäss Anspruch 4 ist.

12. Die landwirtschaftliche Fungizidzusammensetzung gemäss Anspruch 11, in welcher die Benzamidingruppen enthaltende Verbindung ausgewählt ist aus der Gruppe, bestehend aus (1) N,N-Diethyl-N'-phenylthiocarboxybenzamidin, (2) N,N-Diethyl-N'-(p-bromophenyl)thiocarboxybenzamidin und (3) N,N-Diethyl-N'-(p-tert-butylphenyl)thiocarboxybenzamidin.

13. Die landwirtschaftliche Fungizidzusammensetzung gemäss Anspruch 8, in welcher die Verbindung eine Verbindung der allgemeinen Formel (IV) gemäss Anspruch 6 ist.

14. Landwirtschaftliche Fungizidzusammensetzung gemäss Anspruch 13, in welcher die eine Benzamidingruppe enthaltende Verbindung ausgewählt ist aus der Gruppe, bestehend aus (1) N,N-Diethyl-N'-benzylthiocarboxybenzamidin und (2) N,N-Diethyl-N'-benzylthiocarboxy-o-chlorobenzamidin.

**Revendications**

1. Composé du groupe des benzamidines répondant à la formule (I):

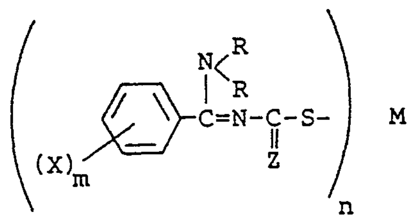

(I)

dans laquelle R représente un groupe alkyle en $C_{1-4}$, X représente un atome d'halogène ou un groupe alkyle en $C_1$—$C_4$; *m* représente un nombre entier, 0, 1 ou 2; Z représente un atome d'oxygène ou un atome de soufre; *n* représente un nombre entier, 1 ou 2; quand *n* vaut 1, M représente un groupe alkyle en $C_1$—$C_4$, un group alcényle en $C_3$—$C_5$, un groupe phényle de formule

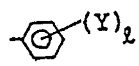

ou un groupe benzyle de formule

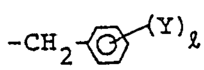

(où Y représente un groupe alkyle en $C_1$—$C_4$, un atome d'halogène, un groupe alkoxy en $C_1$—$C_4$, ou un groupe nitro, et *l* représente un nombre entier, 0, 1, 2 ou 3); et quand *n* vaut 2, M représente un atome d'un métal choisi parmi le nickel, le cobalt et le zinc, à la condition que, quand Z est un atome d'oxygène, M ne soit pas un groupe alkyl en $C_1$—$C_4$.

2. Composé du groupe des benzamidines selon la revendication 1, dans lequel ledit composé répond à la formule générale (II):

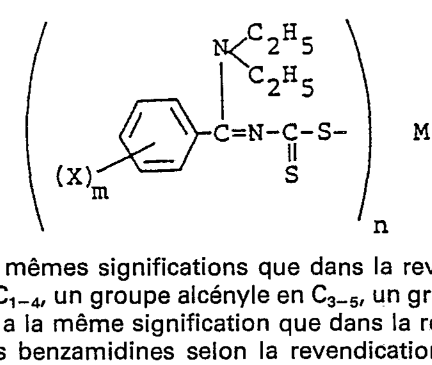

(II)

dans laquelle X, *m* et *n* ont les mêmes significations que dans la revendiction 1 et lorsque *n* vaut 1, M représente un groupe alkyle en $C_{1-4}$, un groupe alcényle en $C_{3-5}$, un groupe benzyle ou un groupe benzyle halogéné, et quand *n* vaut 2, M a la même signification que dans la revendication 1.

3. Composé du groupe des benzamidines selon la revendication 2, ledit composé du groupe des benzamidines étant choisi parmi (1) la N,N-diéthyl-N'-n-propyldithiocarboxybenzamidine, (2) la N,N-diéthyl-N'-benzyl-dithiocarboxybenzamidine, (3) le bis-(N,N-diéthyl-benzamidino-N'-carbodithioate) de

nickel, (4) le bis(N,N-diéthylbenzamidino-N'-carbodithioate) de zinc et (5) le bis-(N,N-diéthyl-benzamidino-N'-carbodithioate) de cobalt.

4. Composé du groupe des benzamidines selon la revendication 1, dans lequel ledit composé répond à la formule générale (III):

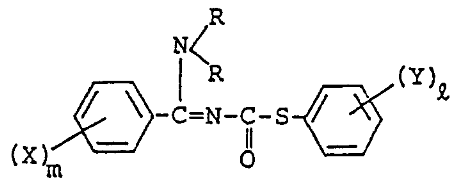

(III)

dans laquelle R, X, *m* et *l* ont les mêmes significations que dans la revendication 1, et Y représente un groupe alkyle en $C_{1-4}$ ou un atome d'halogène.

5. Composé du groupe des benzamidines selon la revendication 4, ledit composé du groupe des benzamidines étant choisi parmi: (1) la N,N-diéthyl-N'-phénylthiocarboxybenzamidine, (2) la N,N-diéthyl-N'-(p-bromophényl)thiocarboxybenzamidine et (3) la N,N-diéthyl-N'-(p-tert-butylphényl)thiocarboxybenzamidine.

6. Composé du groupe des benzamidines selon la revendication 1, ledit composé répondant à la formule générale (IV)

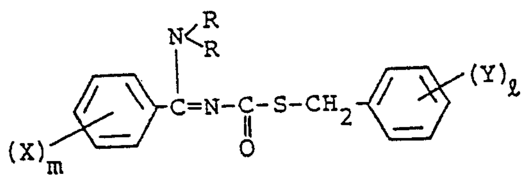

(IV)

dans laquelle R, X, *m* et Y ont les mêmes significations que dans la revendication 1 et *l* est un nombre entier 0, 1 ou 2.

7. Composé du groupe des benzamidines selon la revendication 6, ledit composé du groupe des benzamidines étant choisi parmi (1) la N,N-diéthyl-N'-benzylthiocarboxybenzamidine et (2) la N,N-diéthyl-N'-benzylthiocarboxy-o-chlorobenzamidine.

8. Composition fongicide pour l'agriculture, qui comprend à titre d'ingrédient actif un composé du groupe des benzamidines représenté par la formule générale (I) de la revendication 1.

9. Composition fongicide pour l'agriculture selon la revendication 8, dans laquelle ledit composé répond à la formule générale (II) de la revendication 2.

10. Composition fongicide pour l'agriculture selon la revendication 9, dans laquelle ledit composé du groupe des benzamidines est choisi parmi (1) la N,N-diéthyl-N'-n-propyldithiocarboxybenzamidine, (2) la N,N-diéthyl-N'-benzyl-dithiocarboxybenzamidine, (3) le bis-(N,N-diéthylbenzamidino-N'-carbodithioate) de nickel, (4) le bis (N,N-diéthylbenzamidino-N'-carbodithioate) de zinc et (5) le bis-(N,N-diéthyl-benzamidino-N'-carbodithioate) de cobalt.

11. Composition fongicide pour l'agriculture selon la revendication 8, dans laquelle ledit composé répond à la formule générale (III) de la revendication 4.

12. Composition fongicide pour l'agriculture selon la revendication 11, dans laquelle ledit composé du groupe des benzamidines est choisi parmi: (1) la N,N-diéthyl-N'-phénylthiocarboxybenzamidine, (2) la N,N-diéthyl-N'-(p-bromophényl)thiocarboxybenzamidine et (3) la N,N-diéthyl-N'-(p-tert-butylphényl)thiocarboxybenzamidine.

13. Composition fongicide pour l'agriculture selon la revendication 8, dans laquelle ledit composé répond à la formule générale (IV) de la revendication 6.

14. Composition fongicide pour l'agriculture selon la revendication 13, dans laquelle ledit composé du groupe des benzamidines est choisi parmi (1) la N,N-diéthyl-N'-benzylthiocarboxybenzamidine et (2) la N,N-diéthyl-N'-benzylthiocarboxy-o-chlorobenzamidine.